# EUROPEAN PATENT APPLICATION

(11) **EP 4 613 240 A1**
(43) Date of publication of application: **10.09.2025**
(21) Application number: 24305349.3
(22) Date of filing: 07.03.2024
(51) Int. Cl.: A61F 2/00

(54) **IMPLANTABLE MESH INTRODUCER INCLUDING A CAPE AND METHODS THEREOF**

(71) Applicant: Sofradim Production, 01600 Trevoux (FR)
(72) Inventor: BAILLY, Pierre, 01600 TREVOUX (FR); BRUNE, Thierry, 01600 TREVOUX (FR)
(74) Representative: Germain Maureau

(57) **Abstract**

The present disclosure describes an implantable mesh introducer including at least a cape configured to cover an implantable mesh and a handle, as well as methods and surgical kits associated therewith.

## Description

### Technical Field

The present disclosure describes an implantable mesh introducer, and particularly an implantable mesh introducer including a cape configured to cover the mesh during introduction into a patient and release the mesh inside the patient.

### Background

In laparoscopic and/or robotic surgery, one or more trocars may be required to allow passage of surgical tools, such as surgical graspers, scissors, fasteners, lights, and/or optical systems therethrough, as well as to maintain intra-abdominal pressure, e.g., insufflation, in a patient's abdomen to ensure workspace for the surgery (pneumoperitoneum).

Trocars may have different diameters to accommodate the passage of such surgical tools, as well as implants also having variable diameters or sizes. Generally, trocars commonly used may be 5-15 mm diameter trocars for laparoscopic or robotic surgery. Particularly, 5, 10, 12, or 15 mm diameter trocars may be used in traditional laparoscopy and 8 and 12 mm diameter trocars may be used in robotic surgery.

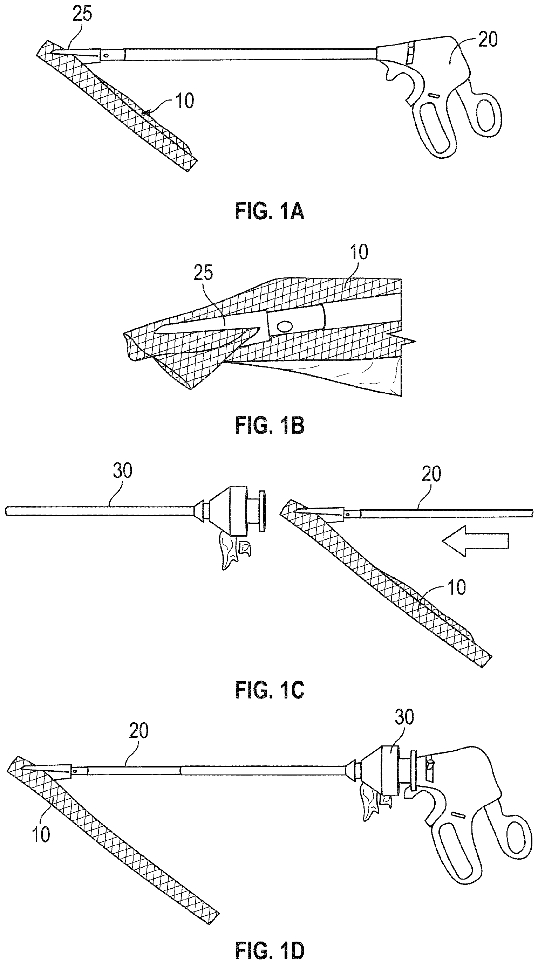

As shown in Figs. 1A-1D, for some surgeries, it may be necessary to introduce an implantable surgical mesh 10 through a trocar 30 via a surgical tool 20, such as a surgical grasper. As best shown particularly in Fig. 1A, the surgical tool 20 may include a tool handle having a surgical arm extending therefrom and including a pair of jaw members 25 extending therefrom. The jaw members 25 may be used to directly pinch a distal end portion of the implantable mesh 10 for introduction into a patient. As further shown in Figs. 1C and 1D, the pinching of the mesh 10 by the jaw members 25 is maintained throughout insertion into a patient. Essentially, a portion of the surgical tool 20 is passed through the trocar 30 with the jaw members 25 tightly pinched against the mesh 10 and dragging the mesh 10 through the trocar 30 under pressure. The combination of pinching and dragging of the mesh 10 by the tool 20 can damage the mesh 10, such as by cutting, stretching, or denting the mesh 10. In addition, because the mesh 10 is uncovered prior to and during insertion, the mesh 10 can be easily contaminated any time prior to complete insertion.

Also, the external diameter of the tool 20, e.g., a surgical grasper, has to be smaller than the internal diameter of the trocar 30 to be able to leave room for the rolled or folded mesh 10 for both the mesh 10 and the tool 20 to pass through the trocar 30. For example, a surgical tool 20 including a 5 mm external diameter may be used to introduce a mesh 10 having less than about a 5mm diameter when using a 10 mm diameter trocar.

Difficulty can arise when trying to pass a mesh through a trocar which is too small for the mesh alone or with the surgical tool. Because the mesh size is dependent on the size of the hernia defect and thus not easily reduceable in size, the alternative is typically to replace the trocar with a larger trocar which takes time, increases cost, and produces a larger hole in the patient's abdominal wall which is associated to an increased risk of developing an incisional hernia later on.

It is an object of the present disclosure to provide an implantable mesh introducer of reduced size to provide more room or volume inside the trocar for the mesh, as compared to conventional surgical graspers. The need to change trocars based on the large size of an introducer and/or mesh can also be reduced and/or avoided.

It is another object of the present disclosure to provide an implantable mesh introducer configured to cover and protect the mesh from damage and/or contamination prior to insertion.

It is still another object of the present disclosure to provide an implantable mesh introducer configured to insert a mesh without mechanically fixing (e.g., grasping, pinching, stitching, clipping, tying, adhering, etc.) the mesh to any portion of the introducer thereby reducing the likelihood of stressing and/or damaging the mesh prior to insertion, as compared to conventional surgical graspers.

It is still another object of the present disclosure to provide an implantable mesh introducer that includes a removable and/or adjustable handle. The removable and/or adjustable handle allows the mesh introducer to vary in length longitudinally to accommodate mesh of different sizes.

### SUMMARY

Implantable mesh introducers are described herein. The introducers may include at least a push stick, a cape, and a handle. The handle is detachably coupled to the proximal end portion of the push stick. The cape is connected to a distal end portion of the push stick. The cape extends between a free proximal end portion and fixed distal end portion. The cape is intended to surround at least a majority, if not an entirety, of an implantable mesh during insertion of the mesh into a patient.

In some embodiments, a cape connector is positioned on a distal end portion of the push stick. The cape connector is configured to attach the fixed distal end portion of the cape to the distal end portion of the push stick.

In some embodiments, the handle is an elongate handle.

In some embodiments, a handle connector is positioned on a proximal end portion of the push stick. The handle connector removably connects a distal end portion of the handle to a proximal end portion of the push stick. The handle connector and the distal end portion of the handle are configured to matingly engage with each other to removably couple the handle connector to the handle.

In some embodiments, the handle connector may be a male (or female) luer connector and the distal end portion of the handle may include a complimentary female (or male) luer connector. In some embodiments, the handle connector and the distal end portion of the handle are configured to be removably attached via at least one of a threaded configuration, a friction fit, or a snap fit.

The handle may define an elongate rod having a thickness or an outer perimeter larger than a thickness or an outer perimeter of the push stick. In some embodiments, the elongate rod may further include an open channel extending along a longitudinal axis of the elongate rod. A portion of the push stick being configured to frictionally fit within the open channel of the elongate rod.

In some embodiments, the elongate rod may further include a plurality of open connector cavities defined within the elongate rod. The open connector cavities being in communication with the open channel. The handle connector is configured to frictionally fit within each of the open connector cavities to removably attach the push stick to the handle. In some embodiments, the elongate handle includes three open connector cavities evenly spaced across the elongate handle.

A grip member may be positioned on the proximal end portion of the elongate rod. In some embodiments, the grip member is an ergonomic grip member including a plurality of finger grooves spaced along a length of a first side of the ergonomic grip member and a single finger groove positioned on a second side of the ergonomic grip member opposite the first side, to receive fingers of a user of the introducer ergonomically. The ergonomic grip member may display a curved profile.

In some embodiments, the grip member is a cross-shaped grip member including a first branch perpendicular to a second branch. The handle connects to approximately a center of the cross-shaped grip member where the first branch and the second branch intersect. One or both of the first branch and the second branch of the cross-shaped grip member may include a plurality of finger grooves defined in an outer edge of the branches.

In some embodiments, the grip member is a mushroom-shaped grip member including a stem part and a cap part. The stem part extends proximally from the proximal end portion of the elongate handle along the longitudinal axis of the handle. The cap part extends generally perpendicular to the longitudinal axis of the elongate handle and is positioned proximal to the stem part. The stem part widens proximally from the elongate handle to the cap part defining an inclined surface. The inclined surface includes at least one circumferential indentation configured to secure a retainer ring thereto.

In some embodiments, the mesh introducer further includes at least one retainer ring or retainer tube. The retainer ring and retainer tube is configured to slide along an exterior of the cape during introduction of the cape and mesh through a trocar. A retainer opening is defined through the retainer ring or retainer tube for passing the cape and mesh through the retainer ring or retainer tube.

In some embodiments, the retainer ring includes a frustoconical shape. In some embodiments, the entire retainer ring is a frustoconical shape. In some embodiments, the retainer ring includes a frustoconical proximal portion and a pillar distal portion.

The retainer ring or the retainer tube may include a slot defined through an exterior of the retainer ring or retainer slot. The slot is in communication with the retainer opening along an entire length of the retainer ring or retainer tube.

In some embodiments, a distal end portion of the retainer ring or the retainer tube includes a guide part which is wider than a proximal end portion of the retainer ring or the retainer tube. The guide part being configured to be seated on and surround an exterior of a trocar for proper placement of the introducer onto the trocar.

The retainer tube extends a length greater than the retainer rings. The retainer tube extends a length sufficient to prevent the push stick from buckling in use of the introducer to introduce a mesh into a patient. In some embodiments, the retainer tube defines a length generally equal to a length of the push stick.

In some embodiments, the push stick is a rigid metal push stick and the cape is a flexible polymeric cape.

Surgical kits including at least one mesh introducer are also provided herein. The surgical kits may further include one or more of an implantable mesh or a trocar. Methods of delivering an implantable mesh into a patient are also provided.

### BRIEF DESCRIPTION OF THE DRAWINGS

Various embodiments of the kits and/or components are described herein with reference to the drawings wherein:
Figs. 1A-1D are side views of a surgical tool and mesh of the prior art;
Fig. 2A is a schematic side view of an implantable mesh introducer including a cape as described in at least one embodiment herein;
Fig. 2B is a cross-sectional view of the implantable mesh introducer of Fig. 2A as described in at least one embodiment herein;
Figs. 2C-2D are schematic side views of the implantable mesh introducer of Fig. 2A as described in at least one embodiment herein;
Fig. 3A is a perspective view of an implantable mesh introducer including a handle as described in at least one embodiment herein;
Fig. 3B is an expanded view of the cape connector of the introducer of Fig. 3A as described in at least one embodiments herein;
Fig. 3C is an expanded view of the handle removably attached to a handle connector of the implantable mesh introducer depicted in Fig. 3A as described in at least one embodiment herein;
Fig. 3D is a perspective view of the handle of Fig. 3C separated from the handle connector as described in at least one embodiment herein;
Figs. 4A and 4B are each a side perspective view from opposite sides, of a grip member of an implantable mesh introducer as described in at least one embodiment herein;
Fig. 5 is a perspective view of a grip member of an implantable mesh introducer as described in at least one embodiment herein;
Fig. 6A is a perspective view of a mesh introducer including an adjustable handle as described in at least one embodiment herein;
Fig. 6B is a side view of the handle of the mesh introducer of Fig. 6A as described in at least one embodiment herein:
Fig. 6C is an expanded view of the adjustable handle removably attached to a handle connector of the implantable mesh introducer depicted in Figs. 6A-6B as described in at least one embodiment herein;
Figs. 6D and 6E are each a cross-sectional view transverse to a longitudinal axis of the introducer along a connector cavity and a channel, respectively, as described in at least one embodiment herein;
Fig. 7A is a perspective view of a retainer ring as described in at least one embodiment herein;
Fig. 7B is a cross-sectional view of the retainer ring of Fig. 7A as described in at least one embodiment herein;
Fig. 7C is a cross-sectional view of the grip member of Fig. 6B in combination with one or more retainer rings of Fig. 7A as described in at least one embodiment herein;
Figs. 8A-8C are side views of a method of using a mesh introducer including a cape and at least one retainer ring as described in at least one embodiment herein;
Fig. 9 is a side view of a retainer ring as described in at least one embodiment herein;
Fig. 10 is a perspective view of a retainer ring as described in at least one embodiment herein;
Figs. 11A and 11B include perspective views of a retainer ring alone and engaged with a trocar, respectively, as described in at least one embodiment herein;
Fig. 12 is a perspective view of a mesh introducer including an adjustable handle and a retainer tube as described in at least one embodiment herein; and,
Figs. 13A-13D are side views of a method of using a mesh introducer including a cape and at least one retainer tube as described in at least one embodiment herein.

### DETAILED DESCRIPTION

The present disclosure describes a mesh introducer configured to deliver at least one implantable mesh into a patient, and particularly into a patient via a trocar. The mesh introducers described herein include at least a push stick having a cape attached on one end and an elongate handle attached on an opposite end. The cape is configured to wrap around some, a majority, or an entirety of an implantable surgical mesh, and particularly an implantable surgical mesh in a compacted configuration, i.e., a folded and/or rolled implantable mesh.

In various embodiments, the cape includes a thin sheet of polymeric material sufficient for wrapping around and/or unwrapping from being around an implantable mesh for delivery into a patient's body. Suitable cape materials display a coefficient of friction sufficient that is low enough to limit the forces between the trocar and the introducer reducing stress to the mesh during insertion. Suitable cape materials also display a high tensile strength allowing the cape to be very thin thereby reducing the volume inside the trocar provided by introducer. Some non-limiting examples of suitable polymeric materials include, but are not intended to be limited to, high-density polyethylene, ultra-high molecular weight polyethylene, polyether ether ketone (PEEK), polyamides, polytetrafluoroethylene (PTFE), polyethylene terephthalate (PET), polypropylene, and combinations thereof. In some embodiments, the cape is made of high-density polyethylene.

In some embodiments, the cape can be made of a transparent material allowing the mesh to be visually observed through the cape, prior to, during, and/or after insertion into a patient. A transparent cape allows the medical personnel to easily see a state of the mesh, such as to determine whether the mesh is damaged and/or contaminated in some manner, without having to remove the mesh from the cape. This can be especially useful for identifying such issue prior to insertion or at least before implantation.

The cape may have a thickness less than or equal to about 100 microns. In some embodiments, the thickness of the cape may range from about 0.1 to about 50 microns. In some embodiments, the thickness of the cape may range from about 1 to about 25 microns. In some embodiments, the thickness of the cape may range from about 5 to about 15 microns. In some embodiments, the thickness of the cape may be about 10 microns.

In addition to being thin, the cape is designed to display have a lower coefficient of friction when slid across the inside of the trocar than the mesh being slid directly across the inside of the trocar without the cape at least partially positioned therebetween.

Because the cape is thin and flexible, the cape can receive, cover, and/or wrap around, an implantable mesh arranged in any suitable configuration, i.e., compacted mesh, folded mesh, rolled mesh, etc. The capes (and/or introducers) described herein may be used with any implantable surgical mesh.

By implantable, the surgical mesh described herein is configured to be positioned at a location within a patient's body for any sufficient amount of time to at least temporarily treat and/or repair a soft tissue defect, such as a hernia or prolapse.

In the context of this application the term "mesh," "surgical mesh," or "implantable mesh" refers to an arrangement of biocompatible filaments or yarns (e.g., a knitted fabric, a woven fabric, or a nonwoven fabric) arranged in a manner to include pores within the mesh face that can encourage tissue ingrowth. The mesh can be bioresorbable, partially bioresorbable or non-bioresorbable. The mesh includes first and second opposite faces and an outer perimeter generally defining a center of the mesh on each face. The mesh can also be provided in any shape (rectangular, square, circular, oval, etc.) and size. Such meshes are well known to persons skilled in the art.

In some embodiments, the implantable mesh is a two-dimensional knitted fabric. In some embodiments, the implantable mesh is e a three-dimensional knitted fabric. In some embodiments, the implantable mesh may be a self-fixating mesh (e.g., ProGrip^{®} mesh by Medtronic). In some embodiments, the mesh may include one or more layers of fabric and may optionally include an anti-adhesion barrier layer positioned on at least one portion or one side of the fabric thereby forming a composite mesh.

The introducer may also include a push stick to which the cape is temporarily or permanently connected. The cape may also be configured to wrap around at least a portion, if not a majority or all, of the push stick.

The push stick can be made of any material suitable for passing the cape, alone or with a surgical mesh wrapped therein, into or out of a patient's body via trocar or opening in a patient's body. Some non-limiting examples of suitable materials used to form the push stick include, but are not intended to be limited to, metal such as stainless steel, silver, gold, titanium, aluminum, and the like, or a polymeric material such as a polycarbonate, polyoxymethylene, polypropylene, polyethylene terephthalate glycol, carbon fiber, and the like, as well as combinations thereof. While the push stick may include a wide variety of materials within the scope of the present disclosure, in some embodiments, the push stick is a rigid push stick made of a metal and/or a polymeric material. The material is sufficiently rigid to provide a good pushing force into a patient without buckling or failing, while maintaining a thickness or diameter small enough to allow the introducer to, with a mesh therein, pass through a trocar of selected size. It is advantageous generally for the material to be of sufficient rigidity to provide sufficient pushing force, without buckling or failing, to allow the push stick to be designed to have a thickness or diameter that is as small as possible. Smaller thicknesses or diameters (e.g., outer diameters (OD)) of the push stick reduce the volume that the introducer will take up inside the trocar when introducing a mesh. And smaller push sticks generally allow a user to use the introducer with smaller trocars, and so with a larger variety of trocars, including optional use with trocars that are larger than permissible with the introducer.

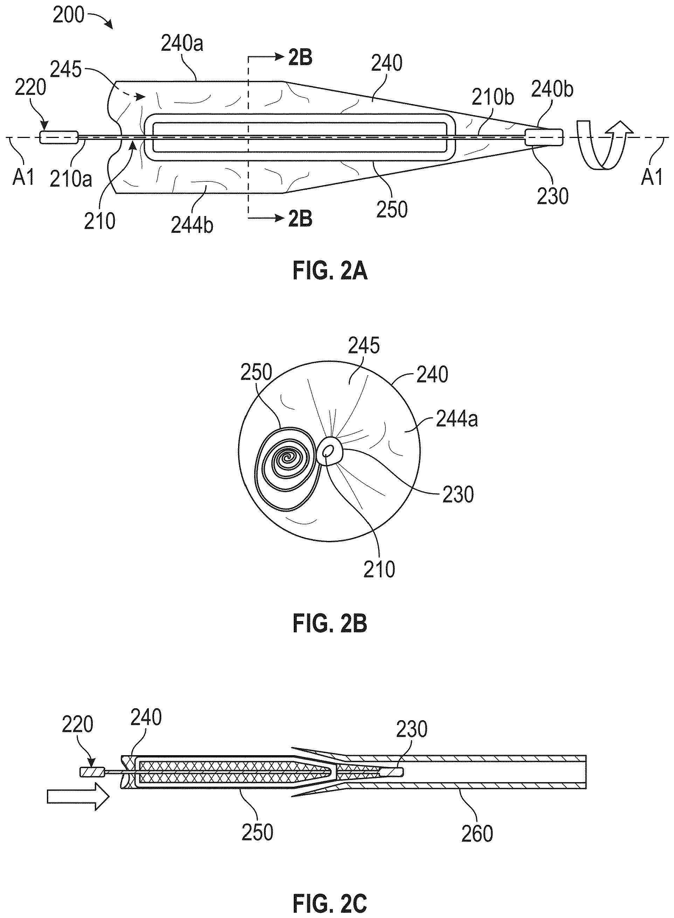

Turning to Figs. 2A-2D, one or more embodiments are depicted of an implantable mesh introducer 200 as described herein, as well as one or more methods of introducing an implantable mesh 250, via the introducer 200, into a patient through a trocar 260. The depicted introducer 200 includes at least a push stick 210 and a cape 240 secured thereto. The push stick 210 defines a longitudinal axis A₁ extending between a proximal end portion 210a and distal end portion 210b. The proximal end portion 210a of the stick 210 may include a handle connector 220. The distal end portion 210b of the stick 210 may include a cape connector 230. The handle connector 220 is configured to attach a handle (not shown in FIGS. 2A-2D) to the push stick 210. It is contemplated, the handle connects directly to the push stick 210- i.e., without need of a handle connector 220. The cape connector 230 is configured to attach the cape 240 to the push stick 210 generally opposite the handle.

The cape 240 extends between a proximal end portion 240a and a distal end portion 240b. The distal end portion 240b of the cape 240 is secured to the distal end portion 210b of the stick 210 by the cape connector 230. In a contemplated embodiment, the cape 240 connects directly to the push stick 210- i.e., without need of a cape connector 230.

The proximal end portion 240a of the cape 240 is free of and/or unsecured to the push stick 210. The cape 240 may extend proximally from the cape connector 230, or distal end portion 210b of the push stick 210, along a portion of the longitudinal axis A₁ of the stick 210 defining a pocket 245 configured to receive the implantable mesh 250 therein. The mesh 250 may be received in the pocket 245 in a compacted form, i.e., folded, rolled, etc. In some embodiments, at least a portion, which may be a majority or an entirety, of a longitudinal length of the push stick 210 may also be received within the pocket 245.

As best seen in Fig. 2B, the mesh 250 remains free of the push stick 210, i.e., the mesh 250 is not rolled or folded directly around the push stick 210. Rather, the folded or rolled mesh 250 is compacted prior to being combined with the introducer 200 and is secured inside the pocket 245 between the push stick 210 and the cape 240.

In some embodiments, the cape connector 230 permanently secures the cape 240 to the push stick 210. Some non-limiting examples of permanent cape connectors include one or more of glues, adhesives, rivets, pins, screws, welding, and the like.

After inserting the implantable surgical mesh 250 into the cape 240, i.e., into pocket 245, the cape 240 may be wrapped around the mesh 250. For example, the stick 210 may be rotated, such as via the handle connector 220, in a clockwise (or counter- clockwise) direction to wrap the cape 240 around the mesh 250 inside the pocket 245, as indicated by the arrow of Fig. 2A and further depicted in Figs. 2B and 2C.

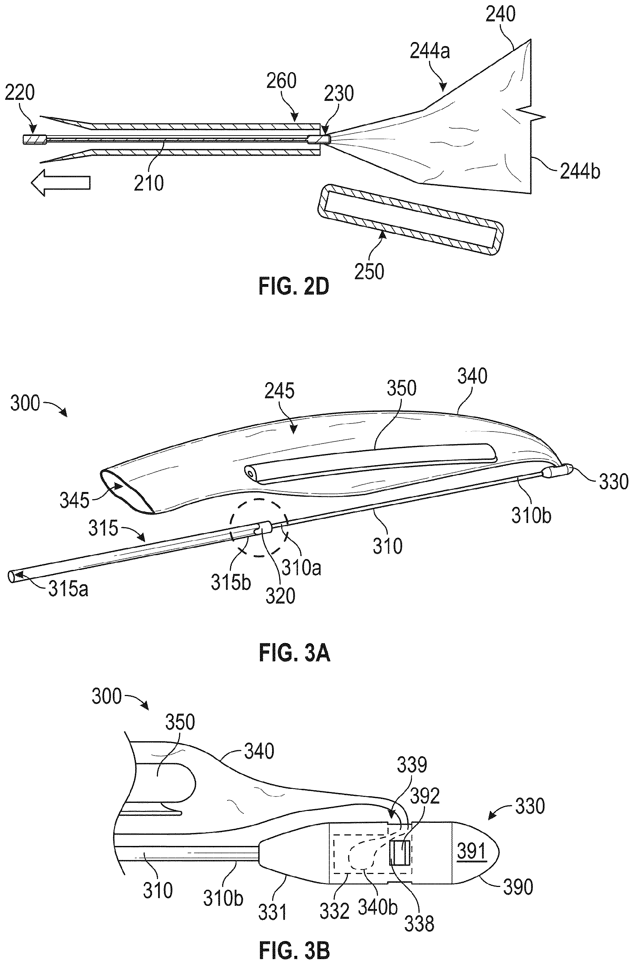

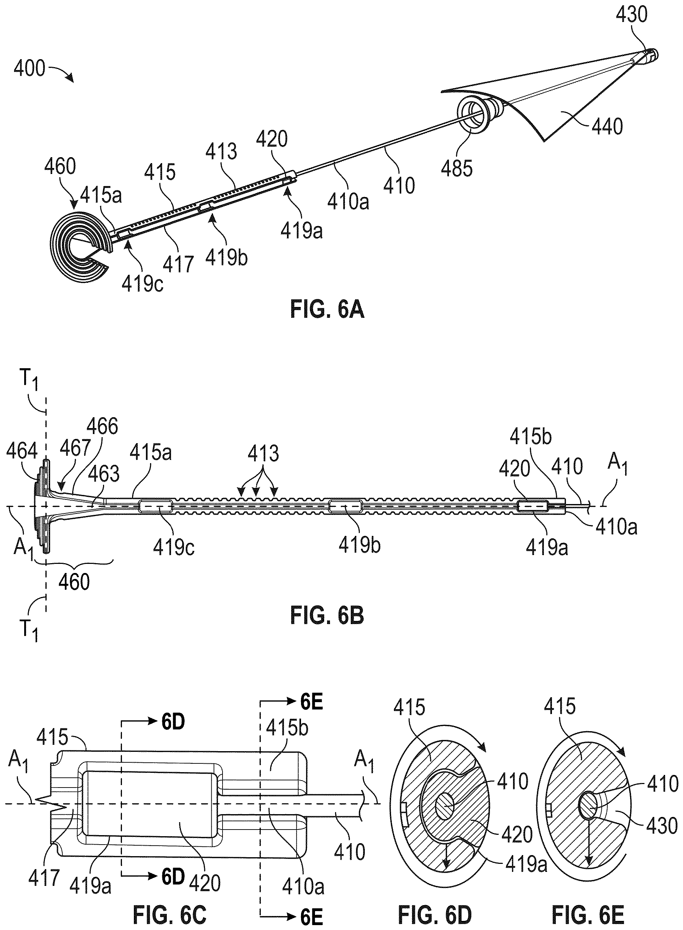

As depicted in Fig. 2C, the cape 240 and the mesh 250, in the wrapped configuration, may be moved distally (as indicated by an arrow) through a trocar 260, into a patient near or at the site of implantation of the mesh 250. In some embodiments, the handle connector 220 of the introducer 200 is manipulated by medical personnel, directly or indirectly via an intermediate surgical tool, to pass the cape 240 and mesh 250 completely through the trocar 260 into the patient. In alternative embodiments, as shown in Figs. 3A and 6A, the handle connector 220 is temporarily, i.e., removably, attached to an elongate handle 315, 415 for manipulation by medical personnel, directly or indirectly, to pass the cape and mesh completely through a trocar into a patient.

As depicted in Fig. 2D, once the cape 240 is free of the restraints of the wall of the trocar 260, the cape 240 at least partially inverts unwrapping the mesh 250 and freeing the mesh 250 of the cape 240. In some embodiments, at least a majority, if not an entirety, of the cape 240 inverts inside the patient. In some embodiments, the cape 240 naturally or automatically inverts upon exiting the trocar 260 inside the patient, and particularly following a moving of the introducer 200, i.e., push stick 210, in a proximal direction back through the trocar 260 away from the patient.

As further illustrated in Figs. 2A-2D, in some embodiments, the cape 240 is designed to transition between an expanded configuration (Figs. 2A-2B), an intermediate wrapped configuration (Fig. 2C), and a delivery configuration (Fig. 2D). In the expanded configuration, the cape can be at its widest or at least sufficiently wide to receive the mesh therein prior to wrapping. In the wrapped configuration, both the cape and the mesh are at their narrowest or at least sufficiently narrower than the expanded configuration to be passed through a trocar into a patient. In the delivery configuration, the cape can be sufficiently inverted and/or unwrapped to allow the mesh to be free of the introducer inside the patient.

As particularly depicted in Fig. 2D, in the inverted delivery configuration, at least a portion, if not a majority or entirety, of an interior surface 244a of the cape 240 becomes an exterior surface of the cape 240 while the exterior surface 244b becomes an interior surface of the cape 240. In the inverted configuration, at least a portion, if not a majority or entirety, of the cape 240 extends distally from cape connector 230.

In some embodiments, the cape can be in an unwrapped configuration inside the patient by rotating, particularly via the handle connector or handle, in a counterclockwise (or clockwise) direction to unwrap the cape around the mesh. The unwrapped configuration occurs prior to inversion of the cape.

In some embodiments, the cape naturally transitions to an unwrapped configuration after exiting the trocar inside the patient thereby releasing the mesh.

Figs. 3A-3D include various depictions of an introducer 300 including a push stick 310, an elongate handle 315, handle connector 320, a cape connector 330, and a cape 340. Particularly, Fig. 3A depicts a mesh introducer 300 including a cape 340 (in an expanded configuration) secured to a distal end portion 310b of a push stick 310 by the shaped cape connector 330. An implantable surgical mesh 350 in a compact configuration, i.e., rolled, and/or folded, is located in a pocket 345 defined by the cape 340.

Fig. 3B focuses in on the connection between the cape connector 330, the distal end portion 310b of the push stick 310, and the cape 340. In some embodiments, the introducer 300 includes a two-piece cape connector 330 having a base part 331 and a cover part 390. The base part 331 of the cape connector 330 is permanently attached to a distal end portion 310b of the push stick 310. The base part 331 defines an inner cavity 332 (shown in phantom) configured to receive a distal end portion 340b (shown in phantom) of the cape 340 therein. The base part 331 includes one or more base locking recesses 338 defined through the outer wall of the base part 331. The base part 331 also includes a base opening 339 defined through the outer wall of the base part 331 to allow the cape 340 to exit the base part 331. The cover part 390 includes a cover body 391 and one or more cover locking tabs 392 extending proximally therefrom. The one or more cover locking tabs 392 are configured to matingly engage the one or more base locking recesses 338 from inside the inner cavity to form the cape connector 330.

In some embodiments, the base part 331 and the cover part 390 are configured to simply snap together after the distal end portion 340b (shown in phantom) of the cape 340 is positioned within the inner cavity 332 (shown in phantom) with the cape 340 sticking out of the base opening 339. The distal end portion 340b (shown in phantom) of the cape 340 can be twisted, rolled, folded, and/or tied into a cape knot to form a bulbous cape part prior to being inserted into the inner cavity 332 of the connector 330.

The base locking recesses 338 completely pass through the base part making at least a portion of the locking tabs 392 accessible from an exterior of the connector for easy removal. For example, in some embodiments, the locking tabs 392 can be pinched or pressed on through the locking recess 338 from an exterior of the connector 330 to free the locking tabs 392 from the recess 338 thereby separating the base part 331 from the cover part 390). Although depicted as a two-piece or multi-piece design, the cape connector may alternatively be of one piece design.

Fig. 3A further depicts the mesh introducer 300 including a removable elongate handle 315 secured to a proximal end portion 310a of the push stick 310 via the shaped handle connector 320. The elongate handle 315 is designed to increase the overall length of the introducer 300 to ensure the cape 340 and mesh 350 can be fully introduced into a patient and to further ensure the push stick 310 can be easily retrieved from inside the patient after delivery of the mesh 350. In addition, the elongate handle 315 can be helpful in rotating the push stick 310 during wrapping and/or unwrapping of the cape 340 around the mesh 350.

In some embodiments, the elongate handle 315 is a generally linear rod configured to extend further along the longitudinal axis A₁ of the push stick 310 (and/or handle connector 320). The elongate handle 315 may define a length equal to or greater than a length of the push stick 310. The elongate handle 315 may define a thickness greater than a thickness of the push stick 310. The elongate handle 315 may define a stiffness greater than a stiffness of the push stick 310.

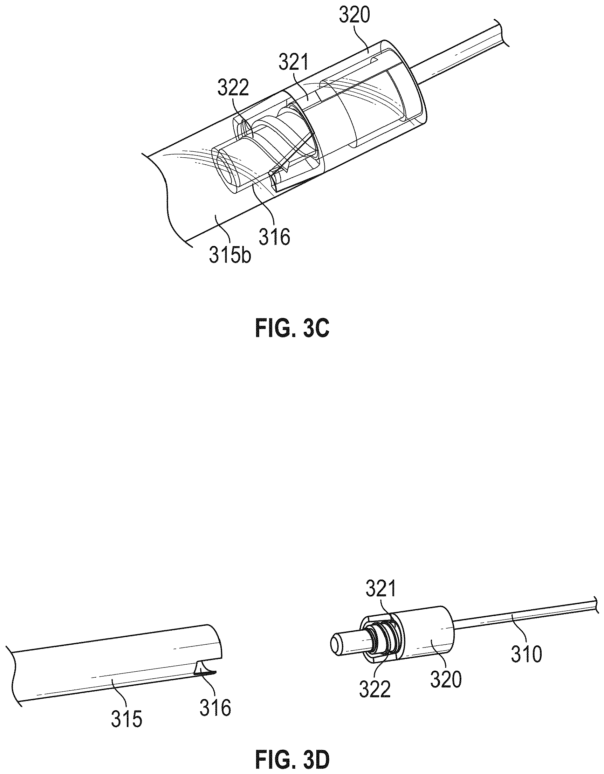

Fig. 3C focuses in on the removable connection between the shaped handle connector 320 and a distal end portion 315b of the elongate handle 315 of Fig. 3A. In some embodiments, the handle connector 320 includes or defines a first coupling member 321 and the distal end portion 315b of the handle 315 may include or define a second coupling member 316. The first and second coupling members 321, 316 are configured to matingly engage with each other to removably couple the handle connector 320 to the handle 315. For example, in some embodiments, the first coupling member 321 is a male (or female) luer connector and the second coupling member 316 is a complimentary female (or male) luer connector. In some embodiments, the first and/or second coupling members 321, 316 are threaded 322.

Fig. 3D illustrates that in some embodiments the handle connector 320 and the elongate handle 315 are configured to be coupled and/or uncoupled as needed without damaging either of the handle 315 or the connector 321. By being removably coupled, the introducers described herein, in some embodiments, are designed to removably connect to multiple interchangeable handle designs having different characteristics (e.g., thickness, length, shape, stiffness, handling, color, etc.).

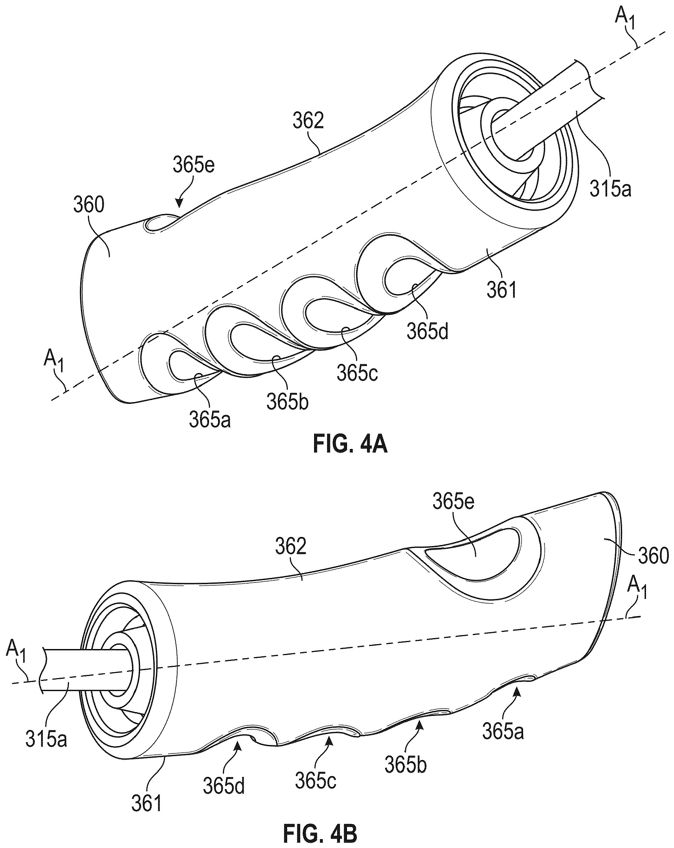

As shown in Figs. 4A-5, the mesh introducer 300 of Figs. 3A-3D may further include a grip member 360 positioned on the proximal end portion 315a of the elongate handle 315. The grip member 360 may be attached to the handle 315 using any suitable method. For example, in some embodiments, the grip member 360 can be designed to be removably coupled to the proximal end portion 315a of the elongate handle 315 in a manner similar to the handle and handle connector as described in more detail hereinabove.

As depicted in Figs. 4A-4B, in some embodiments, the grip member 360 is ergonomically designed for better handling by an operator of the introducer 300. For example, the grip member 360 displays a curved grip body relative to the longitudinal axis A₁ of the introducer 300. The curved grip body defines a curved profile along a length of the grip member 360. The ergonomical grip member 360 also includes a plurality of finger grooves 365a-e defined into an exterior of the grip member 360. Specifically, a first set of finger grooves 365a-d are spaced along a length of a first side 361 of the grip member 360 and a single finger groove 365e is positioned on a second side 362 of the grip member 360 opposite the first side 361. The finger grooves 365a-e are not only useful in moving (i.e., pushing or pulling) the introducer 300 proximally and/or distally along the longitudinal axis A₁, but are also useful in rotating the introducer 300 around the longitudinal axis A₁ for rolling and/or unrolling the cape 340.

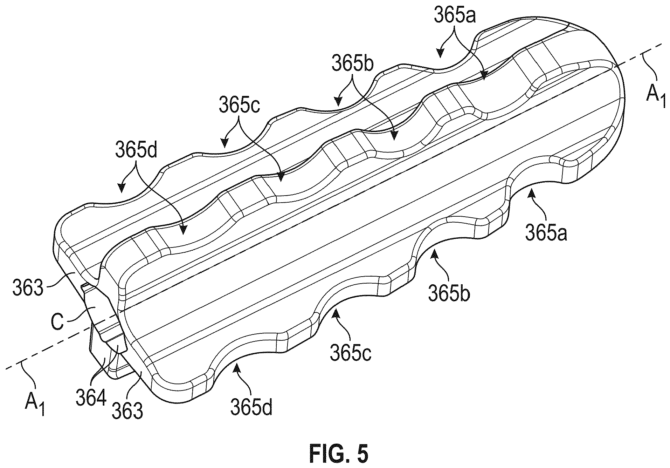

As depicted in Fig. 5, the grip member 360 of the introducer 300 alternatively can be a cross-shaped grip member 360 including a first branch 363 generally perpendicular to a second branch 364. The handle 315 attaches to a center C of the cross-shaped grip member 360 where the first and second branch 363, 364 intersect and/or cross-over. Although the outer edges of the first and second branches 363, 364 narrow in a proximal direction (and/or widen in a distal direction), the cross-shaped grip member 360 generally displays a linear profile extending along the longitudinal axis A₁ of the introducer 300. The cross-shaped grip member 360 also includes a plurality of finger grooves 365a-d defined into the outer edges of each of the first and second branches 363, 364. The finger grooves 365a-d are not only useful in moving (i.e., pushing or pulling) the introducer 300 proximally and/or distally along the longitudinal axis A₁, but may also be useful in rotating the introducer 300 around the longitudinal axis A₁ for rolling and/or unrolling the cape 340.

Figs. 6A-6E include various depictions of a mesh introducer 400 including a push stick 410, an elongate handle 415, a grip member 460, a handle connector 420, a cape connector 430, a cape 440 and at least one retainer ring 485. As provided herein, the mesh introducer 400 is designed to deliver an implantable surgical mesh (not shown in Figs. 6A-6E) in a compacted configuration into a patient. As further provided herein, the cape 440 is designed to wrap around at least the mesh for protection against damage, snags, contamination, friction, etc. prior to and/or during insertion into a patient.

The handle 415 of the introducer 400 is removably coupled to the proximal end portion 410a of the push stick 410 via the handle connector 420. The handle 415 of the introducer 400 is designed to make an overall length of the introducer 400 adjustable without changing the handle 415. The handle connector 420 can simply be connected to a different portion of the handle 415 to change the overall length of the introducer 400 as needed..

As shown in Figs. 6A-6E, the elongate handle 415 is a generally linear rod including an open channel 417 defined therein. The open channel 417 is designed to receive and retain at least a portion of the push stick 410 therein. The push stick 410 is designed to frictionally fit within the open channel 417. The open channel 417 extends along a longitudinal axis A₁ of the elongate handle 415. In some embodiments, the open channel 417 may extend the entire length of the handle 415.

As further shown in Figs. 6A-6D, the elongate handle 415 also includes a plurality of open connector cavities 419a-c defined therein. Each connector cavity 419a-c is designed to receive and retain the handle connector 420 therein. The handle connector 420 is designed to frictionally fit within any of the open connector cavities 419a-c to removably attach the push stick 410 to the handle 415.

Each connector cavity 419a-c, like the channel 417, is open on one side (i.e., the same side) of the handle 415. The side opening design allows the handle connector 420 to be moved in a direction transverse to the longitudinal axis A₁ (e.g., into and out of the page of Figs. 6B and 6C) to enter and/or exit any of the open connector cavities 419a-c to attach to or separate from the handle 415. The side opening design also allows the push stick 410 to be moved in a similar direction transverse to the longitudinal axis A₁ (e.g., into and out of the page of Figs. 6B and 6C) to enter and/or exit the open channel 417 to attach to or separate from the handle 415.

The transverse movement of the handle connector 420 and/or the push stick 410 will require enough force to overcome the friction that maintains the connector 420 in one of the cavities 419a-c and the push stick 410 in the channel 417. Longitudinal movement (e.g., distally and/or proximally) along the longitudinal axis A₁ of the introducer 400 does not separate the handle 415 from the connector 420 or the push stick 410.

As further depicted in Figs. 6A and 6B, the connector cavities 419a-c are spaced intermittently along a length of the handle 415 from each other. Neighboring connector cavities 419a-419b, 419b-419c are connected to and in communication with the open channel 417. In some embodiments, the handle 415 includes three or more open connector cavities 419a-419c equally spaced along a length of the handle 415.

As still further depicted in Figs. 6A and 6B, in some embodiments, the elongate handle 415 includes a plurality of ridges 413 defined in an outer surface of the handle 415. The ridges 413 add a texture to the outer surface along a length of the handle 415 to improve the handling characteristics and gripping ability of the handle 415.

Fig. 6C focuses on the connection between the handle connector 420, the push stick 410, the first connector cavity 419a, the open channel 417, and the handle 415. Particularly, the handle connector 420 is shown frictionally fit within the first open connector cavity 419a with the proximal end portion 410a of the push stick 410 frictionally fit within a portion of the open channel 417. The handle 415 of Fig. 6A-6E, unlike the handle 315 of Fig. 3A, is directly attached to both the handle connector 420 and at least the proximal end portion 410a of the push stick 410.

As shown in Fig. 6D and 6E, the elongate handle 415 may generally define a c-shaped cross-section. More specifically, the elongate handle 415 may define a first c-shaped cross-section along the open channel 417 (Fig. 6E) and a second different c-shaped cross-section along the one or more open connector cavities 419a-c (Fig. 6D). The first c-shaped cross-section being larger circumferentially (outer arrows) and/or thicker radially (inner arrows) than the second c-shaped cross-section.

As further illustrated in Figs. 6A-6B, in some embodiments, the handle 415 includes a grip member 460 positioned on a proximal end portion 415a of the handle 415. The grip member 460 defines a mushroom-shaped cross-section including a distal portion defining a stem part 463 of the grip member 460 and a proximal portion defining a cap part 464 of the grip member 460. The distal stem part 463 of the grip member 460 extends generally along the longitudinal axis A₁ of the handle 415 and push stick 410. The cap part 464 extends generally perpendicular to the longitudinal axis A₁ along transverse axis T₁. The cap part 464 forms a butt end of the introducer 400 suitable for moving (i.e., pushing, pulling, sliding, etc.) the introducer 400 forward or backward along the longitudinal axis A₁ in a proximal or distal direction.

The stem part 463 defines an inclined outer surface 466 which widens in a proximal direction from the elongate handle 415 to the cap part 464 (and/or narrows in a distal direction from the cap part 464 to the handle 415). The inclined surface 466 may include at least one circumferential indentation 467 defined therein. The indentation 467 is configured to temporarily receive a portion of a retaining ring 485 therein.

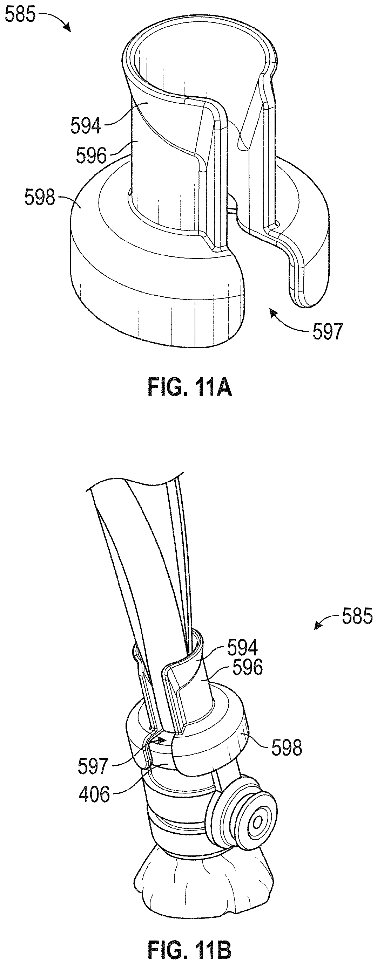

In some embodiments, the open channel 417 of the handle 415 also extends through the grip member 460 defining a c-shaped stem part 463 and a c-shaped cap part 464. In some embodiments, as shown in Fig. 11, the open channel does not extend through the grip member and/or the cap part.

As further illustrated in Fig. 6A, the mesh introducers 400 may also include one or more retainer rings 485. The retainer ring 485 is configured to slide longitudinally along the introducer 400 and over the cape 440 and any compacted mesh inside the cape 440 prior to and during introduction of the cape 440 and mesh into a patient.

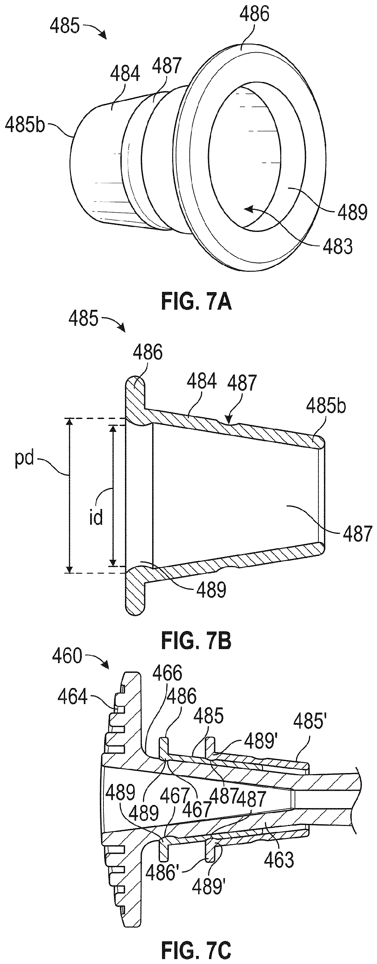

In some embodiments, as shown in Figs. 7A and 7B, the retainer ring 485 as described herein defines a frustoconical shape. The frustoconical retainer ring 485 includes an outer wall 484 that narrows from a proximal outer rim 486 to a distal end portion 485b. The outer wall 484 defines a generally centered retainer opening 483 through the ring 485. The retainer opening 483 also narrows in a distal direction from the proximal outer rim 486. The inner diameter id of the outer rim 486 is slightly smaller than the proximal diameter pd of the opening 483 which creates an inner tab 489 of the outer rim 486.

As shown in Fig. 7C, the inner tab 489 of a first retainer ring 485 is configured to engage the indentation 467 of the grip member 460 when slid across the indentation 467. As further shown in Fig. 7C, in embodiments wherein the introducer 400 includes a second frustoconical retainer ring 485', the inner tab 489' of the second retainer ring 485' is configured to engage the indentation 487 of the first retainer ring 485 stacking and/or securing the second ring 485' on the first ring 485 on the inclined surface 466 of the grip member 460 of the handle 415. The indentation 487 of the retainer ring 485, like the indentation 467 of the grip member 460, is configured to temporarily receive a retaining ring 485' therein. It is envisioned that any number of retainer rings can be stacked on the introducer.

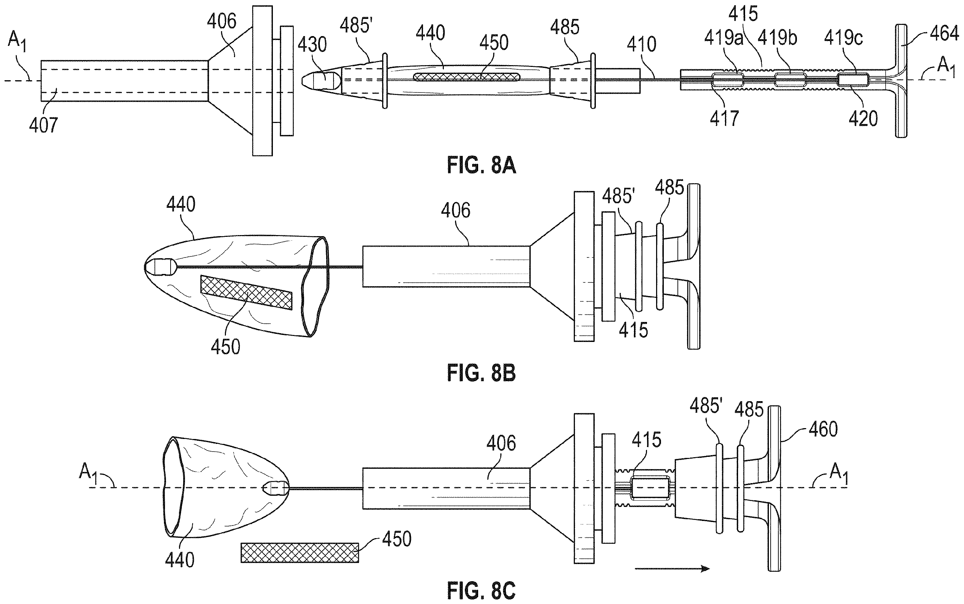

Figs. 8A-8C depict a method of introducing an implantable mesh 450 into a patient through a trocar 406 using the introducer 400 generally of Figs. 6A-6E. Initially, as shown in Fig. 8A, the implantable mesh 450 is shown in a compacted configuration, i.e., folded or rolled, and wrapped inside the cape 440. The cape 440 connected to a distal end portion of the push stick 410 via cape connector 430. A plurality, and particularly a pair, of frustoconical retainer rings 485, 485' surround the outer surface of the cape 440 with the mesh 450 inside the cape 440. The retainer rings 485, 485' being spaced along a length of the introducer 400, including cape 440 and push stick 410. The handle connector 420 on the proximal end portion of the push stick 410 is secured in a third open connector cavity 419c positioned on a proximal end portion of the elongate handle 415 with a portion of the push stick 410 secured in the open channel 417 and passing through a first and second open connector cavities 419a-b.

The mesh introducer 400 can be moved along the longitudinal axis A₁ by pushing on the cap part 464 of the grip member 460 in a distal direction towards trocar 406. Because the handle connector 420 is frictionally fit with the open connector cavity 419c, and/or a portion of the push stick 410 is frictional fit in the open channel 417, the longitudinal movement of the introducer 400 does not separate the handle 415 from the push stick 410. However, because the channel 417 and/or the connector cavities 419a-c are open on one side of the handle 415, movement of the handle connector 420 and/or push stick 410 transverse to the longitudinal axis A₁ (i.e., in and out of the page in Fig. 8A) can separate the connector 420 and/or push stick 410 from the handle 415. The transverse movement will require enough force to overcome the friction that maintains the connector 420 and push stick 410 inside the connector cavity 419c and channel 417, respectively.

As illustrated in Fig. 8B, the retainer rings 485, 485' define an external perimeter (i.e., diameter) larger than a perimeter (i.e., diameter) of the passageway 407 of the trocar 406. The retainer rings 485, 485' are configured to slide over the cape 440 and the mesh 450 during the introduction of the cape 440 and mesh 450 through the passageway 407 of the trocar 406 and into a patient.

As shown in Figs. 8B-8C, the retainer rings 485, 485' are unable to pass through the passageway 407 of the trocar 406 and therefore remain outside the trocar 406. When the handle 415 nears the trocar 406, the retainer rings 485, 485' will continue to slide over the handle 415 and begin to form a stacked configuration (also shown in Fig. 7C) along grip member 460. In the stacked configuration, the retainer rings 485. 485' will remain engaged with the grip member 460 as the introducer 400 is withdrawn from the patient.

As further illustrated in Fig. 8B, once the cape 440 and the mesh 450 are through the trocar 406, the cape 440 can begin to open, either manually via rotation of the introducer 400 or automatically, to release the mesh 450 from the cape 440 inside the patient.

Following the release of the mesh 450 from the introducer 400, e.g., the cape 440, as illustrated in Fig. 8C, the introducer 400, including the handle 415, can be withdrawn from the trocar 406 along the longitudinal axis A₁ by pulling on the cap part 464 of the grip member 460 in a proximal direction as indicated by the arrow.

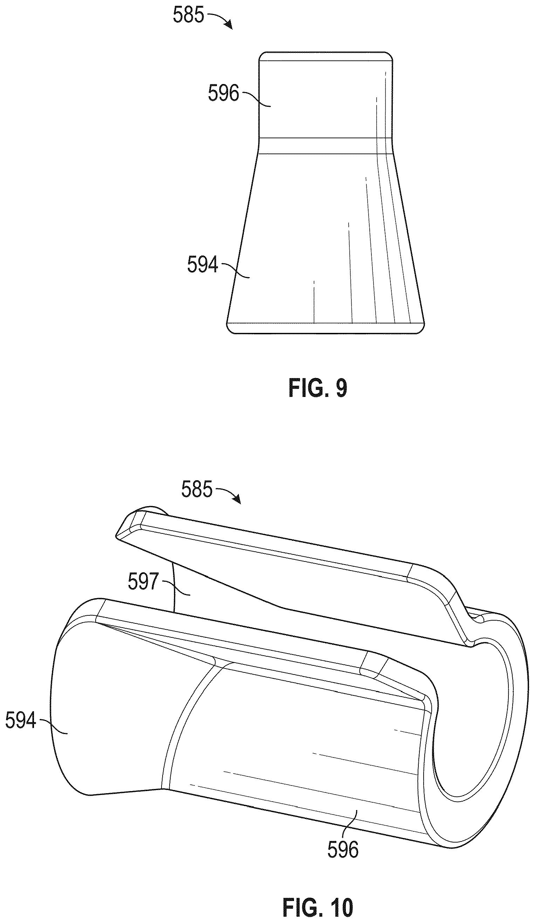

In some embodiments, as depicted in Fig. 9, the retainer ring 585 includes a frustoconical proximal portion 594 and a linear or pillar distal portion 596. The frustoconical proximal portion 594 defines an outer perimeter (e.g., diameter) greater than a perimeter (e.g., diameter) of the trocar passageway to prevent the retainer ring 585 from passing through the trocar. However, in some embodiments, the pillar distal portion 596 defines an external perimeter (i.e., diameter) slightly smaller than the perimeter (i.e., diameter) of the trocar passageway in order to fit at least partially within the trocar passageway. The pillar distal portion 594 can be used as a guide for proper alignment of the retainer ring 585 with the trocar. In some embodiments, as further shown in Fig. 9, the frustoconical portion 594 of the ring 585 is longer in length than the pillar portion 596 of the ring 585.

As depicted in Fig. 10, in some embodiments, the retainer ring 585 further includes an outer slot 597 defined through an entire length of the ring 585. An introducer, with and/or without a mesh wrapped inside a cape, can be passed through the slot 597. For example, after a mesh is introduced into a patient, a user can simply remove the retainer ring radially from the introducer through the slot. In some embodiments, the slotted retainer ring 585 can be removed immediately from the introducer and discarded, as opposed to being stacked on the handle. As further depicted in Fig. 10, in some embodiments, the frustoconical portion 594 of the ring 585 may be shorter than the pillar portion 596 of the ring 585.

As depicted in Figs. 11A and 11B, in some embodiments, the retainer ring 585 includes a frustoconical portion 594, a pillar portion 596 located distal the frustoconical portion 594, a guide part 598 located distal the pillar portion 596, and a slot 597 extending the full length of the ring 585. The guide part 598 is wider than both the frustoconical portion 594 and the pillar portion 596 and defines a generally curved profile. As depicted in Fig. 11B, the guide part 598 is designed to be seated on top of and/or surround an exterior of a trocar 406, as opposed to simply butting up against it and/or entering a portion of the passageway defined in the trocar.

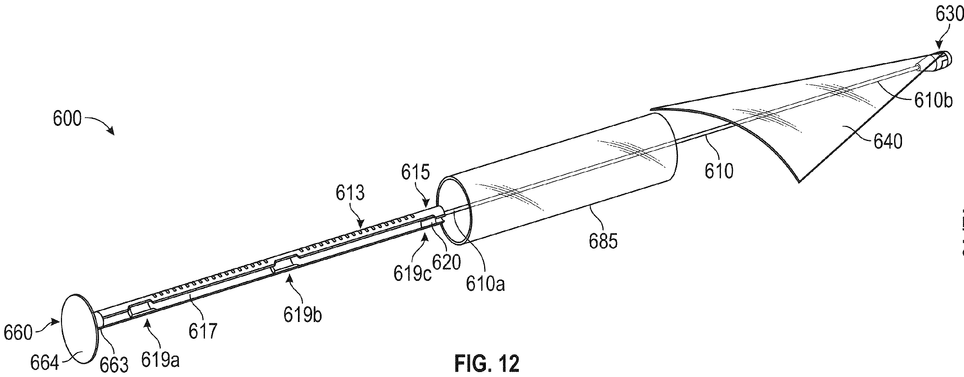

Fig. 12 depicts a mesh introducer 600 including a push stick 610, an elongate handle 615, a grip member 660, a handle connector 620, a cape connector 630, a cape 640 and a retainer tube 685. The elongate handle 615 is removably attached to a proximal end portion 610a of the push stick 610 via handle connector 620. The cape 640 is removably attached to a distal end portion 610b of the push stick 610 via cape connector 630.

The handle 615 includes an open channel 617 extending along an entire length of the handle 615, a plurality of open connector cavities 619a-c intermittently spaced along a length of the handle 615, and a plurality of ridges 613 defined in an outer surface of the handle 615. In some embodiments, the open channel 617 does not extend through the grip member 660.

The retainer tube 685 is configured to slide over a mesh (not shown) located inside the cape 640 during the introduction of the cape 640 and mesh through a trocar and into a patient. The retainer tube 685 is unable to pass through a trocar and remains outside the patient.

The retainer tube 685 also defines an external perimeter (i.e., diameter) larger than an external perimeter (i.e., diameter) of the grip member 660, and particularly an external perimeter (i.e., diameter) of the cap part 664 of the grip member 660. The retainer tube 685 is designed to be removed from the introducer 600 by being passed directly over the handle 615 without separating the handle 615 from the push stick 610 or the grip member 660.

The retainer tube 685 defines a length greater than the retainer ring. In some embodiments, a length of the retainer tube 685 is at least half a length of the push stick 610. In some embodiments, a length of the retainer tube 685 is generally equal to a length of the push stick 610. In some embodiments, a length of the retainer tube 685 is greater than a length of the push stick 610.

The length of the retainer tube can be beneficial for various reasons. For example, the retainer tube covers a large amount of the cape and mesh limiting exposure of the cape and mesh to contamination via direct contact. In another example, the length of the retainer tube, as compared to the retainer rings, provides a larger surface area for a user to grasp for better handling or grip of the introducer during insertion. In yet another example, the length of the retainer tube can limit the amount of flex or bend of the push stick during insertion.

During insertion, a trocar or trocar passageway can provide resistance to the introducer causing the push stick to begin to bend or flex. If the resistance on the introducer is high enough and the introducer does not include a retainer tube, the push stick may flex or bend to the point of deformation of the push stick or separation of the push stick from the handle. The length and outer perimeter of the retainer tube can limit the amount of flex or bend of the push stick to avoid deformation of the push stick or separation of the push stick from the handle during insertion.

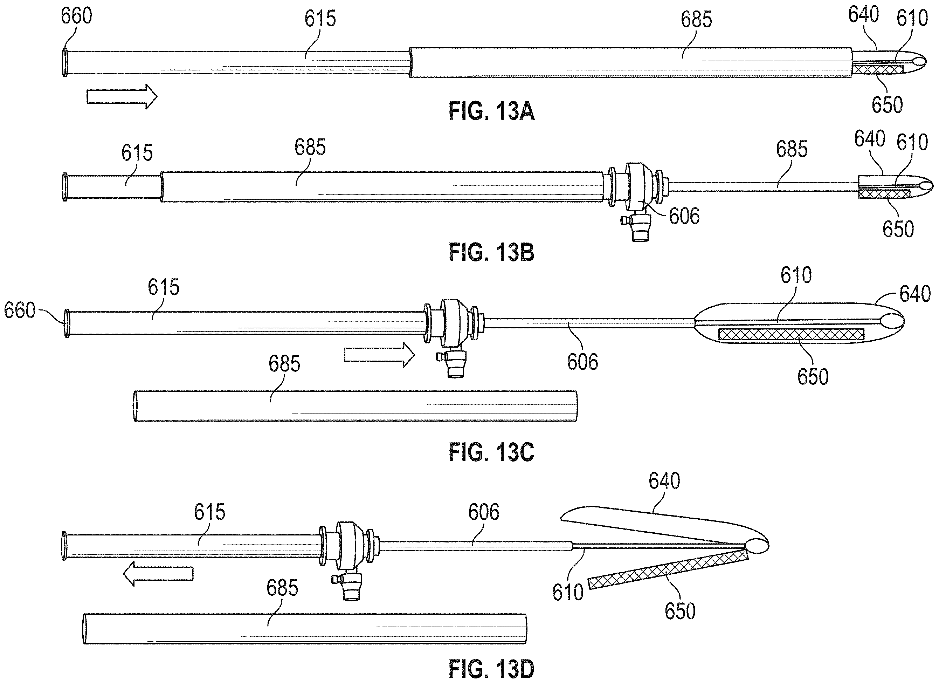

Figs. 13A-13D depict a method of introducing an implantable mesh 650 into a patient through a trocar 606 using the introducer 600 generally of Fig. 12. Initially, as shown in Fig. 13A, an implantable mesh 650, in a compact configuration, is wrapped in a cape 640 of a mesh introducer 600 and retained in a retainer tube 685. The retainer tube 685 covers a majority of the cape 640, the mesh 650, and/or the push stick 610. An elongate handle 615 of the introducer 600 is not covered by the retainer tube 685.

As shown in Fig. 13B, the mesh introducer 600 may be moved longitudinally by pushing the handle 615 in a distal direction, as indicated by the arrow, through a passageway of a trocar 606 to enter a patient (not shown). The retainer tube 685 remaining outside the trocar 606 (and/or patient).

As shown in Fig. 13C, once the mesh 650 is completely between the trocar 606 and the patient, the retainer tube 685 can be removed from the introducer 600 by moving the tube 685 longitudinally in a proximal direction away from the trocar 606 and over the handle 615.

As shown in Fig. 13D, after the mesh 650 is free of the cape 640 inside the patient, the handle 615 can be moved longitudinally in a proximal direction away from the trocar 606 (as indicated by the arrow) to withdraw the introducer 600 from the trocar 606 and leaving the mesh 650 in the patient.

The cape connector, the handle connector, the handle, the grip member, the retainer ring, and/or the retainer tube may be made using any suitable method including but not limited to 3-D printing, injection molding, extrusion, pressing, foaming, and the like.

The cape connector the handle connector, the handle, the grip member, the retainer ring, and/or the retainer tube may also be made from any suitable biocompatible material including but not limited to polymeric materials such as high-density polyethylene, ultra-high molecular weight polyethylene, polyether ether ketone (PEEK), polyamides, polytetrafluoroethylene (PTFE), polyethylene terephthalate (PET), polypropylene, polycarbonate, acrylonitrile butadiene styrene (ABS), and/or metallic materials such as stainless steel, silver, gold, titanium, aluminum, and the like. The cape connector and/or the handle connector may be attached to the push stick using any suitable method including but not limited to over-molding, gluing, extruding, welding, crimping, threading, snap-fitting, and the like.

Surgical kits including any of the mesh introducers described herein are also provided. A surgical kit may include at least one implantable mesh introducer including a cape, a push stick, and a removable and/or adjustable elongate handle. The introducer may further include a grip member and/or one or more retainer rings and/or retainer tubes.

The surgical kits described herein may further include at least one or more of an implantable mesh, a surgical tool, and/or a surgical trocar.

It will be understood that various modifications may be made to the embodiments disclosed herein. Thus, those skilled in the art will envision other modifications within the scope and spirit of the disclosure.

## Claims

1. A mesh introducer comprising:
a push stick extending between a proximal end portion and a distal end portion;
a cape extending between a free proximal end portion and a fixed distal end portion, the fixed distal end portion of the cape secured to the distal end portion of the push stick; and
a handle extending between a proximal end portion and a distal end portion, the distal end portion of the handle configured to detachably couple to the proximal end portion of the push stick.

2. The mesh introducer of claim 1, wherein the cape is attached to the push stick via a cape connector positioned on a distal end portion of the push stick.

3. The mesh introducer of claim 1, wherein the proximal end portion of the push stick comprises a handle connector and the distal end portion of the handle is detachably coupled to the handle connector.

4. The mesh introducer of claim 3, wherein the handle is an elongate handle.

5. The mesh introducer of claim 4, wherein the handle connector includes a male luer connector or a female luer connector, and the distal end portion of the elongate handle includes a complimentary female luer connector or a male luer connector.

6. The mesh introducer of claim 4, wherein the handle connector and the distal end portion of the elongate handle are configured to be removably attached via at least one of a threaded configuration, a friction fit, or a snap fit.

7. The mesh introducer of claim 4, wherein the elongate handle is an elongate rod having a thickness or an outer perimeter larger than a thickness or an outer perimeter of the push stick.

8. The mesh introducer of claim 7, wherein a grip member is positioned on the proximal end portion of the elongate rod.

9. The mesh introducer of claim 8, wherein the grip member is an ergonomic grip member including a plurality of finger grooves spaced along a length of a first side of the ergonomic grip member and a single finger groove positioned on a second side of the ergonomic grip member opposite the first side.

10. The mesh introducer of claim 9, wherein the ergonomic grip member defines a curved profile along a length of the ergonomic grip member.

11. The mesh introducer of claim 8, wherein the grip member is cross-shaped including a first branch perpendicular to a second branch, wherein the handle connects to approximately a center of the cross-shaped grip member where the first branch and the second branch intersect.

12. The mesh introducer of claim 11, wherein each of the first branch and the second branch include a plurality of finger grooves defined therein.

13. The mesh introducer of claim 8, wherein the elongate rod further comprises an open channel extending along a longitudinal axis of the elongate rod, and wherein a portion of the push stick is configured to frictionally fit within the open channel.

14. The mesh introducer of claim 13, wherein the elongate rod further comprises a plurality of open connector cavities defined within the elongate rod, and the handle connector is configured to frictionally fit within one of the open connector cavities to removably attach the push stick to the handle.

15. The mesh introducer of claim 14, wherein the open connector cavities are in communication with the open channel and both the open channel and the open connector cavities are located on the same side of the elongate rod.

16. The mesh introducer of claim 15, wherein the grip member is a mushroom shaped grip member including a stem part and a cap part, wherein the stem part extends proximally along the longitudinal axis of the introducer from the proximal end portion of the elongate handle, and wherein the cap part extends generally perpendicular to the longitudinal axis of the introducer and is positioned proximal the stem part.

17. The mesh introducer of claim 16, wherein the stem part widens proximally from the elongate handle to the cap part defining an inclined surface.

18. The mesh introducer of claim 17, wherein the inclined surface includes at least one circumferential indentation configured to secure a retainer ring thereto.

19. The mesh introducer of claim 1, further comprising a retainer ring configured to slide along an exterior of the cape during introduction of the cape or mesh through a trocar, wherein a retainer opening is defined through the retainer ring for passing the cape and mesh through the retainer ring.

20. The mesh introducer of claim 19, wherein the retainer ring has a frustoconical shape.

21. The mesh introducer of claim 20, wherein the retainer ring further comprises a slot defined through an exterior of the retainer ring, the slot in communication with the retainer opening along an entire length of the retainer ring.

22. The mesh introducer of claim 21, wherein a distal end portion of the retainer ring includes a guide part which is wider than a proximal end portion of the retainer ring, the guide part configured to be seated on and surround an exterior of a trocar for proper placement of the introducer onto the trocar.

23. The mesh introducer of claim 1, further comprising a retainer tube configured to slide along an exterior of the cape during introduction of the cape or mesh through a trocar, wherein a retainer opening is defined through the retainer tube for passing the cape and mesh through the retainer tube.

24. The mesh introducer of claim 23, wherein the retainer tube comprises a length sufficient to prevent the push stick from buckling.

25. A method of delivering an implantable mesh into a patient comprising:
placing a retainer tube over a compacted implantable mesh located inside a cape of a mesh introducer, wherein a distal end portion of the cape is attached to a distal end portion of a push stick of the mesh introducer, and a handle is removably attached to a proximal end portion of the push stick;
pushing the implantable mesh introducer, via the handle, in a distal direction through a passageway of a trocar and into a patient while the retainer tube remains outside the trocar and patient;
withdrawing the retainer tube over the handle in a proximal direction while the compacted implantable mesh is positioned within the trocar or the patient; and
freeing the compacted implantable mesh of the cape inside the patient and pulling the mesh introducer, via the handle, in a proximal direction out of the patient and the trocar.

26. A surgical kit comprising:
a mesh introducer including a cape, a push stick, a removable handle, and at least one of a retainer ring or retainer tube; and
an implantable mesh, a trocar, or both.
